(19) Europäisches Patentamt
European Patent Office
Office européen des brevets

(11) EP 3 124 507 A1

(12) **EUROPEAN PATENT APPLICATION**
published in accordance with Art. 153(4) EPC

(43) Date of publication:
**01.02.2017 Bulletin 2017/05**

(21) Application number: **15768951.4**

(22) Date of filing: **23.03.2015**

(51) Int Cl.:
***C08F 26/02*** *(2006.01)*
***A61L 29/00*** *(2006.01)*
***A61L 31/00*** *(2006.01)*
***C08L 39/00*** *(2006.01)*

(86) International application number:
**PCT/JP2015/058704**

(87) International publication number:
**WO 2015/146897 (01.10.2015 Gazette 2015/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **25.03.2014 JP 2014061804**

(71) Applicants:
- **National University Corporation Yamagata University Yamagata 990-8560 (JP)**
- **NOF Corporation 20-3 Ebisu 4-chome, Shibuya-ku Tokyo 150-6019 (JP)**

(72) Inventors:
- **TANAKA Masaru Yamagata 992-8510 (JP)**
- **NAGASAWA Atsushi Amagasaki-shi Hyogo 660-0095 (JP)**
- **TAKAOKA Toshiaki Amagasaki-shi Hyogo 660-0095 (JP)**
- **AMAYA Naoyuki Tokyo 150-6019 (JP)**

(74) Representative: **Wills, Andrew Jonathan et al Mewburn Ellis LLP City Tower 40 Basinghall Street London EC2V 5DE (GB)**

# (54) POLYMER COMPOUND FOR MEDICAL USE AND COMPOSITION AND DEVICE USING SAME

(57) The present invention provides a polymer compound that can be expected to inhibit activation of a coagulation system, a complement system or a platelet system when brought into contact with in vivo tissue and/or blood since the compound is capable of containing intermediate water, and a composition and a device using the same. This problem is solved by a polymer compound containing a repeating unit represented by general formula (A), and used in contact with in vivo tissue and/or blood, in which in a process in which the polymer compound caused to contain water in a prescribed water content is cooled to -100°C and thereafter heated at a temperature rise rate of 5.0°C/min, a temperature region where exothermicity occurs is present in the range of -80°C or more and -25°C or less, and a temperature region where endothermicity occurs is present in the range of -25°C or more and lower than 0°C:

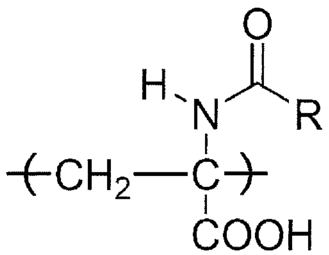

(A)

wherein R represents a hydrocarbon group having 1 to 20 carbon atoms.

EP 3 124 507 A1

**Description**

TECHNICAL FIELD

The present invention relates to a polymer compound suitable for medical use to be used in contact with in vivo tissue or blood, and a composition and a medical device using the same.

BACKGROUND ART

In general, if a biological component such as blood comes into contact with a medical material surface or the like, the material surface is recognized as a foreign matter, and hence, non-specific adsorption, multilayer adsorption or the like, onto the material surface, of a protein or the like contained in biological tissue can occur. Besides, the thus adsorbed biological component can be modified. As a result, a coagulation system, a complement system, a platelet system and the like are activated. Therefore, in order to prevent a surface of a medical material, that is, a contact interface with a living body, from being recognized as a foreign matter, it is desired to impart biocompatibility to the medical material. Specifically, in medical tools such as a heart-lung machine, a dialyzer, a blood storage bag, a platelet storage bag, a blood circuit, an artificial heart, an indwelling needle, a catheter, a guide wire, a stent, an artificial blood vessel and an endoscope, a portion to be brought into contact with a biological material such as blood is desired to have excellent biocompatibility.

As an example of a problem occurring on a medical material surface, a blood filter used in the field of blood transfusion has a problem in which an adverse reaction probably based on the activation of the coagulation system or the complement system is caused in filtration of blood. It is known that production of bradykinin, that is, a representative example of such activation of the coagulation system and the complement system, is triggered by adsorption of coagulation factor XII, that is, a plasma protein, onto the surface of the blood filter, and by contact activity such as modification. As a countermeasure, if the surface of the blood filter can be provided with biocompatibility for inhibiting a damage on a blood component such as the adsorption of the plasma protein onto the surface of the blood filter in the filtration of blood, the activation of the blood (of the coagulation system, the complement system and the platelet system) can be probably inhibited to reduce the adverse reaction occurring in blood transfusion.

Medical materials containing, as a principal component, 2-hydroxyethyl methacrylate (HEMA) or polyvinyl alcohol are conventionally known as medical materials to which a blood component less adheres through contact with blood (Non Patent Literatures 1 and 2), and such materials are used in soft contact lenses as well as artificial vitreous bodies and carriers used in a drug delivery system. Besides, a medical tool including, in a surface portion thereof, a polymer material containing acrylamide as a principal component is known. Furthermore, materials that activate blood at an extremely low degree when brought into contact with blood, such as poly(2-methoxyethyl acrylate) (PMEA) (Patent Literatures 1 and 2), have recently started to be known. Moreover, in consideration that water-soluble poly(ethylene glycol) (PEG) (Non Patent Literatures 3 and 4) has high biocompatibility, for example, a treatment for modifying a surface of a medical material with a PEG chain is performed.

On the other hand, it has not been clarified yet which type, for example in structure, of substance has biocompatibility, and hence, for search for a medical material to be used in contact with a biological component such as blood, a method in which an individual material is actually brought into contact with blood or the like to observe a resultant state is only one established method. On the contrary, owing to recent studies, it has been clarified that there is, in a biocompatible material, a water molecule exhibiting a peculiar behavior designated as "intermediate water".

Figure 1 illustrates a result of measurement, with a differential scanning calorimeter (DSC), of endothermic/exothermic amounts observed when a sample of the PMEA containing 9 wt% of water was cooled to -100°C, and then heated at a rate of 2.5°C/min. As illustrated in Figure 1, if the PMEA containing a sufficient amount of water is cooled to a low temperature and then heated, prescribed exothermicity occurs in a specific temperature region of 0°C or lower (in the vicinity of -40°C in Figure 1), and endothermicity is observed in a wide temperature range from about -10°C to 0°C. It has been revealed, through various examinations, that the exothermicity in the vicinity of -40°C is caused because a part of water molecules contained in the PMEA are ordered, and that the endothermicity occurring at about - 10°C to 0°C is caused because the ordered water molecules are disordered again. In this manner, a water molecule exhibiting a behavior not caused in simple water is present in the water-containing PMEA, and water exhibiting such a behavior is designated as "intermediate water".

It has been revealed that such "intermediate water" is contained in substances having excellent biocompatibility, for example, contained in hyaluronic acid derived from a living body, polysaccharides such as heparin, proteins such as gelatin and albumin, nucleic acids such as DNA and RNA, and the above-described PEG, that is, an artificially synthesized biocompatible material. In this manner, it is presumed "intermediate water" is closely related to development of the biocompatibility in a substance.

The reason why "intermediate water" is generated within a substance is not clear, but it is generated probably

because interaction is caused due to a specific intermolecular force between a polymer chain having high molecular mobility and a water molecule contained in the substance. It is presumed that in a biocompatible material, intermediate water present between a material surface and a hydration shell of a biological component inhibits direct contact therebetween, so as to suppress a foreign body reaction of a living body.

CITATION LIST

NON PATENT LITERATURE

Non Patent Literature 1: "Baiomateriaru no Kiso" (Basic Biomaterials), edited by Kazuhiko Ishihara, Takao Hanawa, Mizuo Maeda, Nihon Igakukan (2011)
Non Patent Literature 2: Tsuruta, T., Contemporary topics in polymeric materials for biomedical applications, "ADVANCED POLYMER SCIENCE", 1996, 126, pp. 1-51
Non Patent Literature 3: Mori, Y.; Nagaoka, S.; Takiguchi, T.; Kikuchi, T.; Noguchi, N.; Tanzawa, H.; Noishiki, Y., A New Antithrombogenic Material with Long Polyethylene Oxide Chains, " Journal of American Society of Artificial Organs", 1982, 28, pp. 459-463
Non Patent Literature 4: Harris JM, ed, Poly(ethylene glycol) chemistry, Biotechnical and Biomedical Application, (U.S.A.), Plenum Press, 1992

PATENT LITERATURE

Patent Literature 1: Japanese Patent Laid-Open No. 2002-105136
Patent Literature 2: Japanese Patent Laid-Open No. 2004-161954

SUMMARY OF INVENTION

PROBLEM TO BE SOLVED BY THE INVENTION

As described above, while it has been revealed that intermediate water present in various substances is closely related to the biocompatibility of the substances, a specific structure of a substance capable of containing intermediate water has not been cleared yet, and as a result, it is still difficult to find a novel substance capable of containing intermediate water.

A problem to be solved by the present invention is to provide a polymer compound that can contain intermediate water, and hence can be expected to inhibit activation of the coagulation system, the complement system or the platelet system otherwise caused through contact with in vivo tissue and/or blood, and to provide a composition and a device using the same.

MEANS TO SOLVE THE PROBLEM

In order to solve the above-described problem, the present invention specifically provides the following:

(1) A polymer compound containing a repeating unit represented by general formula (A), and used in contact with in vivo tissue and/or blood, in which in a process in which the polymer compound caused to contain water in a prescribed water content is cooled to -100°C and thereafter heated at a temperature rise rate of 5.0°C/min, a temperature region where exothermicity occurs is present in the range of -80°C or more and -25°C or less, and a temperature region where endothermicity occurs is present in the range of -25°C or more and lower than 0°C:

[Formula 1]

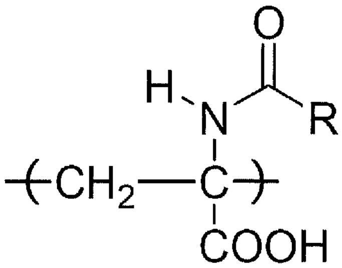

(A)

wherein R represents a hydrocarbon group having 1 to 20 carbon atoms.

(2) The polymer compound according to (1) above, in which R represents an alkyl group having 1 to 3 carbon atoms in the repeating unit represented by general formula (A).

(3) A composition containing the polymer compound according to (1) or (2) above.

(4) A medical device containing the composition according to (3) above in at least a part of a surface thereof.

EFFECTS OF THE INVENTION

A polymer compound of the present invention is capable of containing intermediate water, and is expected to inhibit activation or the like of the coagulation system, the complement system or the platelet system when brought into contact with in vivo tissue and/or blood. When the polymer compound of the present invention is applied, a composition extremely useful as a composition to be used in a medical device used in contact with in vivo tissue and/or blood, such as an artificial kidney membrane, a plasma separation membrane, a catheter, an artificial lung membrane, an endoscope, a filter for extracting in vivo tissue or a protein, an artificial blood vessel or an artificial organ.

BRIEF DESCRIPTION OF DRAWINGS

[Figure 1] Figure 1 is a graph illustrating a result of DSC measurement of water-containing poly(2-methoxyethyl acrylate) (PMEA).

[Figure 2] Figure 2 is a graph illustrating a result of DSC measurement of poly(α-acetamidoacrylic acid) in which an amount of contained water is varied.

[Figure 3] Figure 3 is a diagram of a DSC temperature rise curve measured using a first sample of poly(α-acetamidoacrylic acid).

[Figure 4] Figure 4 is a diagram of a DSC temperature rise curve measured using a second sample of poly(α-acetamidoacrylic acid).

[Figure 5] Figure 5 is a graph illustrating amounts of intermediate water and the like contained in respective samples of an example.

[Figure 6] Figure 6 is a graph illustrating ratios of the intermediate water and the like contained in the respective samples of the example.

DESCRIPTION OF EMBODIMENTS

A polymer compound according to the present invention will now be described in detail.

The polymer compound of the present invention is a polymer compound containing a repeating unit represented by general formula (A) in which R mainly represents an alkyl group having 1 to 20 carbon atoms, and corresponds to what is called α-alkylamidoacrylic acid polymers, and analogs thereof. This group of polymers have a characteristic that they have an electron-withdrawing group and an electro-donating group on the same carbon, and have been conventionally used in hair care products and toiletry products owing to their water absorbency and heat sensitivity.

[Formula 2]

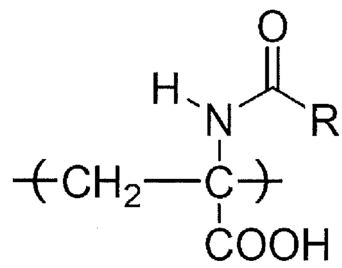

(A)

The present invention was accomplished based on finding that if a group of polymers, in which binding among carbon having an electron-withdrawing group and an electron-donating group forms a main chain, is caused to contain water, intermediate water is unexpectedly generated within the water-containing polymers as described in an example below, and the present invention provides use of the group of polymers in contact with in vivo tissue and/or blood.

Incidentally, a surface of a medical device herein refers to a surface of a material constituting the medical device and a surface portion such as a hole formed in the material to be brought into contact with blood or the like when the medical device is used. Besides, the term "used in contact with in vivo tissue and/or blood" is used herein to inclusively mean an aspect or the like where it is used in contact with the inside of a living body, the skin (also including epidermis and dermis) corresponding to outer surface tissue of a living body, or an eye (cornea). The term "used in contact with in vivo tissue and/or blood" naturally embraces, for example, an aspect where it is used, in a state where it is inserted into a living body or in a state where in vivo tissue is exposed, in contact with the tissue and/or blood, and an aspect where it is used, as a medical material for extracorporeal circulation, in contact with blood corresponding to an in vivo component taken out of a living body.

In the polymer compound of the present invention containing the repeating unit represented by general formula (A) as a principal component, R represents a branched or straight chain hydrocarbon group, such as an alkyl group, an alkenyl group, an alkynyl group, a cycloalkyl group, a cycloalkenyl group or an allyl group, and may be substituted by halogen, nitrogen, phosphorus or sulfur. Besides, the carbon number of R is 1 to 20, preferably 1 to 10, more preferably 1 to 4, and particularly preferably 1 to 3. Among these, a polymer containing one, two or more α-alkylamidoacrylic acid monomers having an alkyl group as R is preferred.

Examples of the α-alkylamidoacrylic acid monomers include, depending on the structure of R, α-acetamidoacrylic acid, α-propylamidoacrylic acid, α-butylamidoacrylic acid, α-isobutyrylamidoacrylic acid, α-pivaloylamidoacrylic acid, α-octylamidoacrylic acid, α-decanoylamidoacrylic acid, α-laurylamidoacrylic acid and α-stearylamidoacrylic acid. Among these specific examples, α-acetamidoacrylic acid, α-propylamidoacrylic acid, α-butylamidoacrylic acid and α-isobutyrylamidoacrylic acid are preferred from the viewpoint of economic efficiency, usability and hydrophilicity. In particular, α-acetamidoacrylic acid, α-propylamidoacrylic acid are more preferred.

Besides, the polymer compound of the present invention can be obtained as a copolymer of any of the α-alkylamidoacrylic acid monomers and a second monomer. Examples of the second monomer copolymerizable with the α-alkylamidoacrylic acid monomer include alkyl acrylamides such as acrylamide, t-butyl acrylamide, n-butyl acrylamide, i-butyl acrylamide, hexyl acrylamide and heptyl acrylamide; dialkyl acrylamides such as N,N-dimethyl acrylamide and N,N-diethyl acrylamide; amino alkyl acrylates such as amino methyl acrylate, amino ethyl acrylate and amino isopropyl acrylate; diamino alkyl acrylates such as diamino methyl acrylate, diamino ethyl acrylate and diamino butyl acrylate; methacrylamide; N,N-dialkyl methacrylamides such as N,N-dimethyl methacrylamide and N,N-diethyl methacrylamide; amino alkyl methacrylates such as amino methyl methacrylate and amino ethyl methacrylate; diamino alkyl methacrylates such as diamino methyl methacrylate and diamino ethyl methacrylate; alkyl acrylates such as methyl acrylate, ethyl acrylate, isopropyl acrylate, butyl acrylate, hexyl acrylate and 2-ethylhexyl acrylate; and alkyl methacrylates such as methyl methacrylate, ethyl methacrylate, butyl methacrylate, hexyl methacrylate and methoxyethyl acrylate.

Any of these second monomers is used in a composition ratio not impairing the intermediate water containing performance of the resultant polymer compound. A content of a monomer providing the repeating unit represented by general formula (A) is preferably about 30 wt% or more, and more preferably 50 wt% or more based on the weight of all monomers contained in the copolymer. If the polymer compound of the present invention is obtained as a copolymer, it may be any of a random copolymer, a block copolymer and a graft copolymer.

If the polymer compound of the present invention is used as a surface treatment agent for a medical device, the polymer compound of the present invention alone may be used, or a composition obtained by mixing with another polymer compound or the like in a composition ratio not impairing the intermediate water containing performance of the polymer compound may be used.

A number average molecular weight of the polymer compound of the present invention can be preferably 10,000 to 500,000, and more preferably 30,000 to 100,000.

Besides, if the polymer compound of the present invention is applied to a medical device used in contact with

in vivo tissue and/or blood, the polymer compound of the present invention may be used to constitute the medical device, or may be used to constitute merely a surface portion of the medical device to be brought into contact with in vivo tissue and/or blood. This is because a material surface structure is in close relation to biocompatibility.

The material and the shape of a base covered with the polymer compound of the present invention are not especially limited, and the base may be in any form such as a porous material, a fiber, a nonwoven fabric, a particle, a film, a sheet, a tube, a hollow fiber or a powder. Examples of the material include natural polymers such as cotton, linen, and hemp, synthetic polymers such as nylon, polyester, polyacrylonitrile, polyolefin, halogenated polyolefin, polyurethane, polyamide, polysulfone, polyethersulfone, poly(meth)acrylate, ethylene-vinyl alcohol copolymers and butadiene-acrylonitrile copolymers, and mixtures of these. Alternatively, examples may include metals, ceramics and composite materials of these, and a plurality of bases may be used together.

A composition containing the polymer compound of the present invention can be used in a medical device used in contact with in vivo tissue and/or blood. Specifically, the composition containing the polymer compound of the present invention is used in at least a part of a surface, to be brought into contact with in vivo tissue and/or blood, of a medical device, such as an implantable artificial organ or treatment instrument, extracorporeal circulation type artificial organs, and catheters (including catheters for circulatory organs such as an angiographic catheter, a guide wire and a PTCA catheter, catheters for digestive organs such as a nasogastric catheter, a gastrointestinal catheter and an esophageal tube, and urological catheters such as a tube, a urethral catheter and a ureteric catheter), and is preferably used over substantially the whole region of the surface to be brought into contact with in vivo tissue or blood.

These medical devices may be further provided with surface lubricity in order to make the devices easily inserted into blood or tissue so as not to damage the tissue. As a method for providing the surface lubricity, a method in which a water-soluble polymer is insolubilized to form a water-absorbing gel layer on a material surface is superior. When this method is employed, a material surface having both the biocompatibility and the surface lubricity can be provided.

The composition containing, as a principal component, the polymer compound of the present invention may be used in a hemostatic, an adhesive for biological tissue, a repairing material for tissue regeneration, a carrier of a drug release system, a hybrid artificial organ such as an artificial pancreas or an artificial liver, an artificial blood vessel, an embolus material, or a scaffolding matrix material for cell culture.

The polymer compound of the present invention is a material having excellent blood compatibility in itself, and various bioactive materials can be supported thereon. Therefore, the polymer compound of the present invention can be used not only in a blood filter but also in various medical devices such as a blood reservoir, a blood circuit, an indwelling needle, a catheter, a guide wire, a stent, an artificial lung device, a dialyzer and an endoscope.

A specific example includes a blood filter in which the polymer compound of the present invention is coated on at least a part of a surface of a base constituting the blood filter. Another example includes a blood bag in which the polymer is coated on at least a part of surfaces, which are to be brought into contact with blood, of the blood bag and a tube connected to the blood bag. As still another example, in an extracorporeal circulation blood circuit that is constituted by a device-side blood circuit part including a tube, an arterial filter, a centrifugal pump, a hemoconcentrator, a cardioplegia and the like, and a surgical field-side blood circuit part including a tube, a catheter, a sucker and the like, at least a part of a surface thereof, to be brought into contact with blood, is coated with the polymer.

Besides, another example includes an indwelling needle assembly in which at least a part of a surface thereof to be brought into contact with blood is coated with the polymer compound of the present invention. This indwelling needle assembly includes an inner needle having an incisive needle tip at the end, an inner needle hub disposed on a base side of the inner needle, a hollow outer needle into which the inner needle can be inserted, an outer needle hub disposed on a base side of the outer needle, a protector attached to the inner needle and movable in an axial direction of the inner needle, and connecting means for connecting the outer needle hub and the protector to each other. Still another example includes a catheter including an elongated tube and an adaptor connected to a base (on a proximal side) in which at least a part of a surface thereof to be brought into contact with blood is coated with the polymer compound of the present invention.

Another example includes a guide wire in which at least a part of a surface thereof to be brought into contact with blood is coated with the polymer compound of the present invention. Further examples include stents in various shapes, such as a hollow tube made of a metal material or a polymer material and having pores on its side surface, or a cylindrical molding formed by knitting a wire of a metal material, a fiber of a polymer material or the like, in which at least a part of a surface thereof to be brought into contact with blood is coated with the polymer compound of the present invention.

Another example includes an artificial lung coated with the polymer compound of the present invention. With respect to such an artificial lung, for example, in an artificial lung of a hollow-fiber membrane external perfusion type in which a large number of porous hollow-fiber membranes for gas exchange are placed in a housing, blood flows on an outer surface side of the hollow-fiber membranes, and an oxygen-containing gas flows inside the hollow-fiber membranes, the outer surfaces or outer surface layers of the hollow-fiber membranes are coated with the polymer compound of the present invention.

Another example includes a dialyzer in which at least a part of a surface thereof to be brought into contact with blood is coated with the polymer compound of the present invention. Such a dialyzer includes, for example, a dialysis fluid circuit including at least one dialysis fluid vessel filled with a dialysis fluid and at least one drainage vessel for collecting the dialysis fluid, and fluid feeding means for feeding the dialysis fluid from a start point of the dialysis fluid vessel to an end point of the drainage vessel, and therefore, it includes a surface to be brought into contact with blood.

A polymerization reaction performed for producing the polymer compound of the present invention is not especially limited in itself, and polymerization can be performed by any of known methods such as radical polymerization, ion polymerization, photopolymerization, and polymerization utilizing a macromer. It is noted that a macromer refers to an oligomer having a polymerizable functional group, a double bond in particular, at an end thereof.

Examples of a method for causing the polymer compound of the present invention to be held on a surface of a medical device include known methods such as a coating method, graft polymerization using radiation, electron beams or ultraviolet, and an introduction method using a chemical reaction with a functional group of a base. Among these methods, especially the coating method is practically preferred because the production operation is easily performed. Examples of the coating method include an applying method, a spraying method and a dipping method, any of which can be employed without restriction. The coat thickness is preferably 0.1 $\mu$m to 1 mm. A coating treatment with the polymer compound of the present invention by the painting method can be practiced, for example, by a simple operation in which a filter medium is dipped in a coating solution obtained by dissolving the polymer compound of the present invention in an appropriate solvent, an excessive portion of the solution is removed and the resultant is air dried. Besides, in order to fix a coating layer more firmly on the filter medium, the coated filter medium may be heated to further improve adhesiveness. Alternatively, the coating layer may be fixed by causing cross-linkage on a surface thereof. For causing the cross-linkage, a crosslinkable monomer may be introduced as a comonomer component. Alternatively, the monomer can be crosslinked with electron beams, $\gamma$-rays or light irradiation.

Examples of the crosslinkable monomer include a compound containing, in one molecule, a plurality of vinyl groups or allyl groups, such as methylene bisacrylamide, trimethylolpropane di(meth)acrylate, triallyl isocyanurate, trimethylolpropane tri(meth)acrylate and pentaerythritol tetra(meth)acrylate, and polyethylene glycol di(meth)acrylate. Among these, polyethylene glycol di(meth)acrylate is preferably used for introducing various functional groups. In this case, an introduction ratio of a compound having a functional group is high, and in addition, a polyethylene glycol chain can be introduced to attain hydrophilicity, and therefore, the non-specific adsorption of unwanted cells and proteins can be suppressed as described above. A molecular weight of the polyethylene glycol chain in this case is preferably 100 to 10,000, and more preferably 500 to 6,000.

As described so far, if the polymer compound of the present invention capable of containing intermediate water or the composition thereof is introduced into at least a part of a surface of a medical tool to be brought into contact with blood, the activation of the coagulation system, the complement system and the platelet system can be inhibited, and hence excellent biocompatibility can be expected to be developed.

In general, it can be checked whether or not a polymer compound or the like is capable of containing intermediate water as follows: The polymer compound or the like is caused to contain water in a prescribed ratio, and through a process in which the resultant is comparatively rapidly cooled to a sufficiently low temperature and then heated to room temperature or higher, the exothermic and endothermic situations of the polymer compound or the like are measured. Specifically, a sample to be checked whether or not it is capable of containing intermediate water is caused to contain water in various amounts of water, the resultant is cooled generally to about -100°C at a cooling rate of about 1 to 10°C/min, subsequently retained at that temperature preferably for about 10 minutes, and then heated generally to about 50°C at a temperature rise rate of about 5.0°C/min, and through this process, if a temperature region where the exothermicity occurs is found in the range of about - 80°C to -25°C, and particularly from about -70°C to -40°C, and a temperature region where the endothermicity occurs is found in the range of about -25°C or more and lower than 0°C, and particularly about -20°C to -5°C, it is regarded that intermediate water is present in the sample. In other words, as a result of various verifications, it is presumed that the exothermicity in the vicinity of -80°C to -25°C is derived from the ordering of water molecules and that the endothermicity in the vicinity of -25°C to 0°C is derived from the disordering of water molecules. Such water molecules making peculiar behavior at a low temperature are designated as "intermediate water" distinguishably from "unfreezable water" that cannot behave as water molecules because constrained by surrounding molecules or "free water" not constrained (or very weakly constrained) by surrounding molecules.

Also a polymer compound or the like capable of containing intermediate water can generally contain "unfreezable water" or "free water". In other words, if a dried polymer compound or the like is caused to contain a slight amount of water, the water is constrained by surrounding molecules to become "unfreezable water", and hence, exothermicity or endothermicity derived from the water molecules does not occur in a cooling or heating process. On the contrary, it is presumed that if a polymer compound or the like capable of containing intermediate water is caused to contain water in a ratio higher than a ratio at which the water can become "unfreezable water", water molecules working as intermediate water are generated within the polymer compound or the like, which probably causes the above-described peculiar temperature history. Besides, it is presumed that if water is contained in a further excessive ratio, water molecules are

not constrained by surrounding molecules, and as a result, "free water" causing exothermicity (in cooling) or endothermicity (in heating) merely at 0°C is generated.

In general, the amounts of "unfreezable water", "intermediate water" and "free water" that can be contained in a polymer compound or the like are determined depending on the type of polymer compound or the like, and in particular, a phenomenon where the ratio of "intermediate water" is reduced owing to an excessive amount of "free water" is also observed. In the present invention, as long as a polymer compound has a water content corresponding to the presence of "intermediate water", the polymer compound can be suitably used even if "intermediate water" is not present therein at another water content.

As described in detail below, a water content of the polymer compound of the present invention is preferably higher than 32 wt%, more preferably 40 wt% or more, and particularly preferably 40 to 60 wt%.

It is noted that the endothermic/exothermic amounts of a sample are generally measured with a differential scanning calorimeter.

EXAMPLES

Now, the present invention will be further described with reference to examples, and it is noted that the present invention is not limited to these examples.

1. Synthesis of α-Acetamidoacrylic Acid

A four-necked flask equipped with a stirrer, a thermometer, a Dimroth condenser and a water-measuring tube was charged with 198.2 g of pyruvic acid (manufactured by Kishida Chemical Co., Ltd.), 66.5 g of acetamide (manufactured by Kishida Chemical Co., Ltd.) and 1.5 L of toluene, and the resultant was refluxed by heating. After refluxing for 6 hours, the resultant was cooled to room temperature. The thus precipitated solid was collected by filtration, and washed with toluene 3 times, resulting in obtaining 110.9 g of a pale yellow solid of α-acetamidoacrylic acid represented by the following formula (B) (yield: 67%).

[Formula 3]

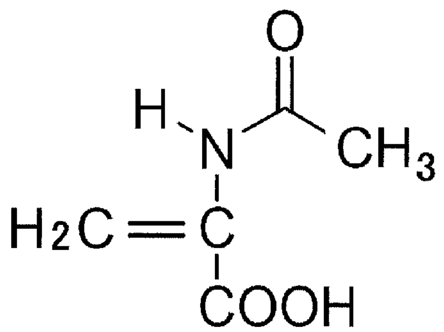

(B)

2. Synthesis of Poly(α-acetamidoacrylic Acid)

A four-necked flask equipped with a stirrer, a thermometer, a Dimroth condenser and a nitrogen introducing tube was charged with 30.0 g of the α-acetamidoacrylic acid synthesized as described above, 1.9 g of 2,2-azobisisobutyronitrile and 130 mL of dimethylformamide (DMF), and the resultant was heated to 60°C. After stirring at 60°C for 20 hours, the resultant was cooled to room temperature. The thus obtained DMF solution of poly(α-acetamidoacrylic acid) was reprecipitated in 1 L of acetone, and washed with acetone 3 times, resulting in obtaining 28.5 g of a white solid of poly(a-acetamidoacrylic acid) (yield: 95%).

The structure of the thus obtained poly(α-acetamidoacrylic acid) was checked through $^1$H-NMR (spectrometer: AL-400 manufactured by JEOL Ltd., measurement frequency: 400 MHz, measurement solvent: DMSO-$d_6$, internal standard: tetramethylsilane ($\delta$ = 0 ppm)), resulting in obtaining a result of $\delta$ = 2.79 ppm, 2H ($CH_2$), 1.85 ppm, 3H ($CH_3CO$). Besides, through IR (IR-Prestige-21 manufactured by Shimadzu Corporation, ATR method), absorption was observed at wavelengths of 2916, 1650, 1510 and 1386 $cm^{-1}$. Furthermore, it was confirmed through molecular weight analysis using GPC (measurement device: HLC-8220GPC, detector: differential refractive index, column: Shodex OHpack SB-803HQ, eluate: 0.1 M NaCl aqueous solution) that the obtained poly(α-acetamidoacrylic acid) had a weight average molecular weight of 39,000.

3. Differential Thermal Analysis of Poly(α-acetamidoacrylic Acid)

A plurality of samples of the poly(α-acetamidoacrylic acid) synthesized as described above were immersed in water to cause the samples to contain different amounts of water. After causing water to be contained, a prescribed amount of each sample was weighed and thinly spread on a bottom of an aluminum pan whose weight had been precedently weighed. The sample was cooled from room temperature to -100°C at a cooling rate of 5.0°C/min, retained for 10 minutes, and then heated from -100°C to 50°C at a temperature rise rate of 5.0°C/min, and during this process, the endothermic/exothermic amounts were measured by obtaining a DSC temperature rise curve using a differential scanning calorimeter (DSC-7000, manufactured by Seiko Instruments Inc.).

Besides, each of the samples was vacuum dried after the DSC measurement by forming a pinhole in the aluminum pan, and an amount of contained water was obtained by measuring the weights before and after the drying. Furthermore, a water content (WC) of each sample was obtained in accordance with the following equation (I):

$$WC = ((W_1 - W_0)/W_1) \times 100 \ldots (I)$$

wherein WC: water content (wt%), $W_0$: dry weight (g) of sample, $W_1$: water-containing weight (g) of sample)

4. Water-containing State of Poly(α-acetamidoacrylic Acid)

Figure 2 illustrates a DSC temperature rise curve of poly(α-acetamidoacrylic acid) caused to contain water at various water contents. Besides, Figures 3 and 4 respectively illustrate DSC temperature rise curves obtained when the water contents were 44.8 wt% and 45.6 wt%. As is obvious from Figure 2, conspicuous endothermicity/exothermicity was not observed in the samples having the water contents of 32 wt% or less during the process of heating from -100°C to 50°C. This is probably because all water molecules contained therein lose freedom, in this range of the water content, due to the interaction with the molecules of poly(α-acetamidoacrylic acid) and hence are in a state of what is called "unfreezable water", and therefore, phenomenons of freezing and melting do not occur.

On the other hand, in the samples having the water contents of about 40 to 60 wt%, exothermicity was obviously observed in the vicinity of -70°C to -40°C, and endothermicity was observed to be caused in the vicinity of -20°C to 0°C. The exothermicity in the vicinity of -70°C to -40°C and the endothermicity in the vicinity of -20°C to 0°C are observed also in polymers conventionally known to contain intermediate water, and seem to correspond to transfer of latent heat caused when a part of the water molecules contained therein are ordered and disordered respectively in these temperature regions. In other words, the water molecules thus behaving are water molecules that do not become unfreezable water although contained in the poly(α-acetamidoacrylic acid) and do not behave as free water owing to some influence from the poly(α-acetamidoacrylic acid) molecules, and are presumed as intermediate water designated distinguishably from these.

Besides, as illustrated in Figure 2, in the samples having the water contents of about 60 wt% or more, endothermicity derived from melting in the vicinity of 0°C caused by free water not constrained by the poly(α-acetamidoacrylic acid) molecules becomes conspicuous, and exothermicity in the vicinity of -70°C to -40°C becomes relatively difficult to observe, but since endothermicity is observed in a range from - 20°C to 0°C, it is presumed that there still exists intermediate water.

Accordingly, the water content of the polymer compound of the present invention is preferably 32 wt% or more, more preferably 40 wt% or more, and particularly preferably 40 to 60 wt%.

In this manner, inside the poly(α-acetamidoacrylic acid) containing water in 32 wt% or more, particularly about 40 wt% or more, movement of latent heat derived from ordering and disordering of a part of the water molecules contained therein in a temperature region of 0°C or lower is observed, and therefore, it was revealed that poly(α-acetamidoacrylic acid) is capable of containing intermediate water.

On the basis of the DSC temperature rise curves observed in the samples of poly(α-acetamidoacrylic acid) containing water in various water contents illustrated in Figures 2 to 4, amounts of unfreezable water, free water and intermediate water at each water content were calculated, and the results are illustrated in Figure 5. Specifically, a total amount of intermediate water and free water contained in each sample was obtained on the basis of the endothermic amount in the vicinity of -20°C to 0°C on the DSC temperature rise curve obtained at each water content. Then, a value obtained by subtracting the thus obtained total amount of intermediate water and free water from the amount of contained water, that is, a difference ($W_1$ - $W_0$) between the dry weight and water-containing weight of each sample measured as described above, was defined as the amount of unfreezable water. Besides, the amount of unfreezable water of each sample was divided by the dry weight ($W_0$) of the sample to obtain a content of unfreezable water per polymer unit weight of each sample, namely, a total contained amount of intermediate water and free water, and the water content obtained

in accordance with the above-described equation (I) was indicated on the abscissa. Incidentally, in the samples having a water content lower than 65 wt%, endothermicity at 0°C was not observed and hence it was presumed that free water was not contained, and therefore, in a region of the water content lower than 65 wt% in Figure 5, the total amount of intermediate water and free water was given as the amount of intermediate water. Furthermore, Figure 6 illustrates ratios among unfreezable water, free water and intermediate water in the total amount of these water contained in each of the poly(α-acetamidoacrylic acid) samples of Figure 5.

As illustrated in Figures 5 and 6, in poly(α-acetamidoacrylic acid), if the water content is about 30 wt% or less, substantially the whole amount of contained water becomes unfreezable water, and if the contained water is increased, the amount of water contained as intermediate water is increased (up to about 60 wt%) while the amount of water contained as the unfreezable water is retained substantially constant, and if the contained water is further increased, water is started to be contained as free water.

In this manner, it was revealed that if poly(α-acetamidoacrylic acid), that is, an α-alkylamidoacrylic acid polymer, is caused to contain water in a prescribed ratio or more, a part of water molecules contained therein becomes intermediate water. If the polymer compound of the present invention, such as poly(α-acetamidoacrylic acid), capable of containing intermediate water, or a composition thereof is introduced into at least a part of a surface of a medical tool, the intermediate water exists between the surface of the medical tool and a hydration shell of a biological component, so as to prevent contact therebetween. Therefore, the polymer compound or the like of the present invention can suppress a foreign body reaction of a living body, and is expected to exhibit biocompatibility.

## Claims

1. A polymer compound comprising a repeating unit represented by general formula (A), and used in contact with in vivo tissue and/or blood, wherein in a process in which the polymer compound caused to contain water in a prescribed water content is cooled to -100°C and thereafter heated at a temperature rise rate of 5.0°C/min, a temperature region where exothermicity occurs is present in the range of -80°C or more and -25°C or less, and a temperature region where endothermicity occurs is present in the range of -25°C or more and lower than 0°C:

[Formula 4]

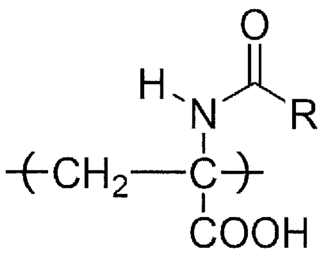

(A)

wherein R represents a hydrocarbon group having 1 to 20 carbon atoms.

2. The polymer compound according to claim 1, wherein R represents an alkyl group having 1 to 3 carbon atoms in the repeating unit represented by general formula (A).

3. A composition comprising the polymer compound according to claim 1 or 2.

4. A medical device comprising the composition according to claim 3 in at least a part of a surface thereof.

FIG. 1

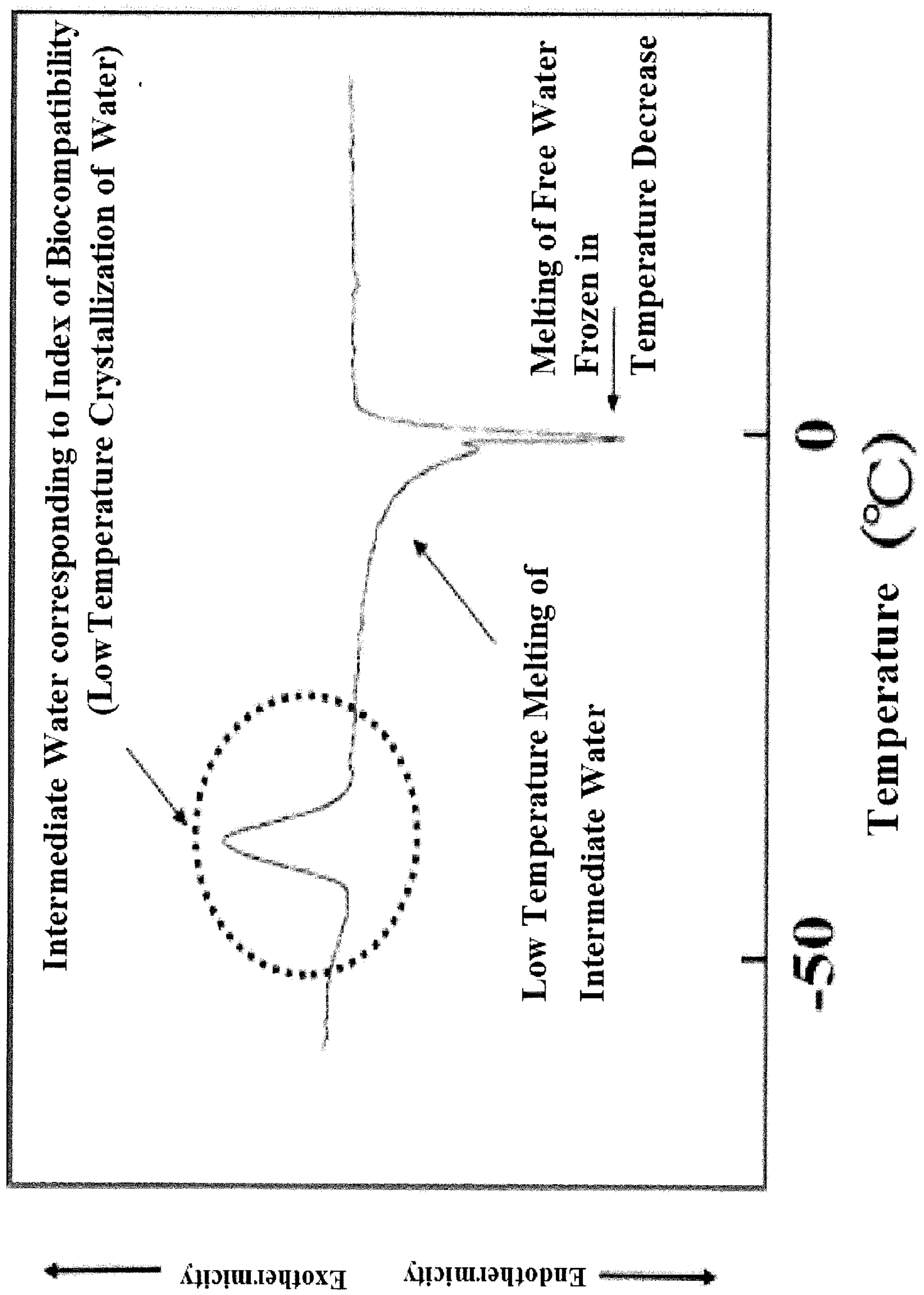
Intermediate Water corresponding to Index of Biocompatibility
(Low Temperature Crystallization of Water)
Melting of Free Water
Frozen in
Temperature Decrease
Low Temperature Melting of
Intermediate Water
-50
0
Temperature (°C)
Exothermicity
Endothermicity

FIG. 2

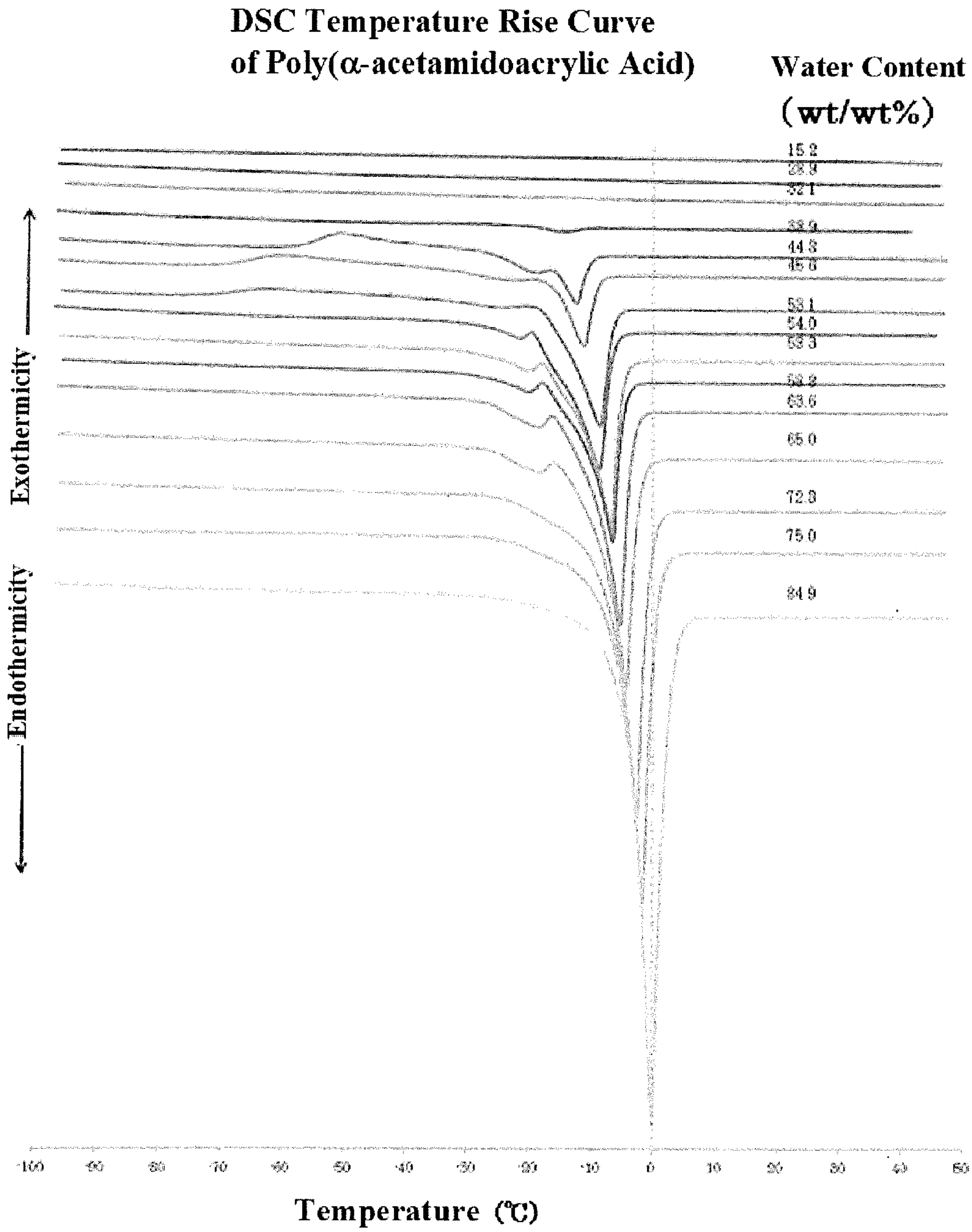
DSC Temperature Rise Curve
of Poly(α-acetamidoacrylic Acid)
Water Content
(wt/wt%)
15.2
44.3
53.1
54.0
63.6
65.0
72.3
75.0
84.9
Exothermicity
Endothermicity
Temperature (℃)

FIG. 3

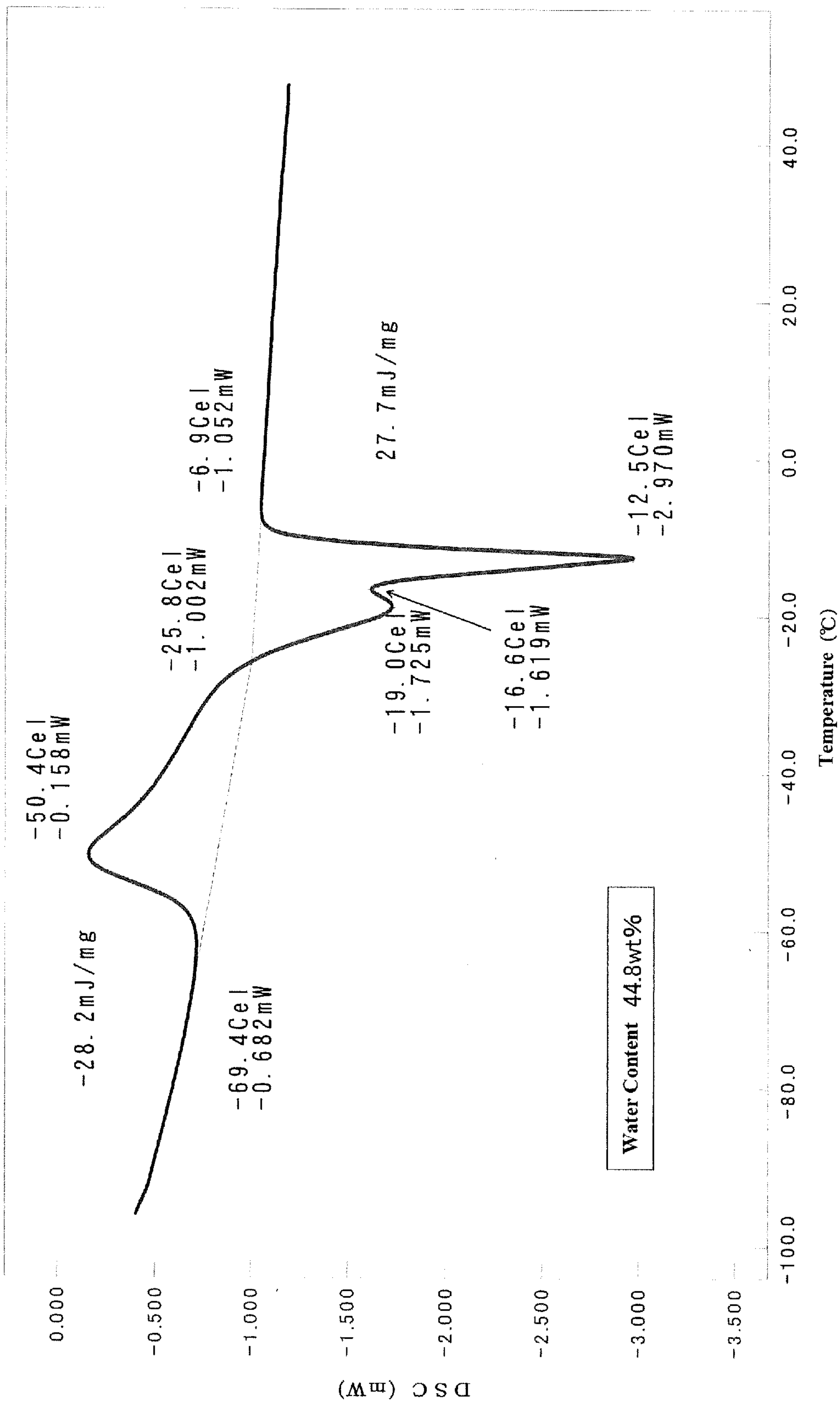
-50.4Cel
-0.158mW
-28.2mJ/mg
-69.4Cel
-0.682mW
-25.8Cel
-1.002mW
-6.9Cel
-1.052mW
27.7mJ/mg
-19.0Cel
-1.725mW
-16.6Cel
-1.619mW
-12.5Cel
-2.970mW
Water Content 44.8wt%
0.000
-0.500
-1.000
-1.500
-2.000
-2.500
-3.000
-3.500
DSC (mW)
-100.0
-80.0
-60.0
-40.0
-20.0
0.0
20.0
40.0
Temperature (℃)

FIG. 4

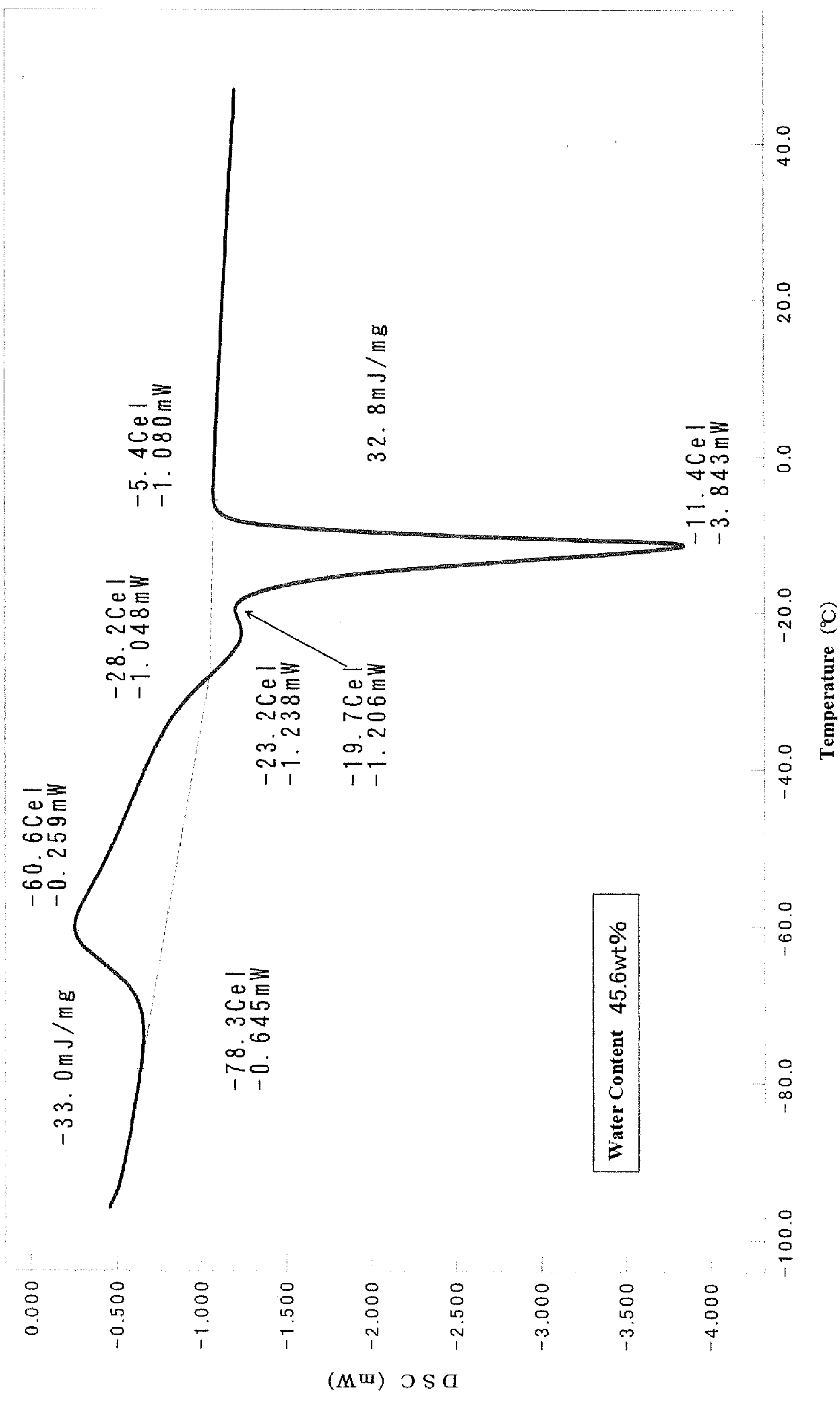

-33.0mJ/mg
-60.6Cel
-0.259mW
-78.3Cel
-0.645mW
-28.2Cel
-1.048mW
-23.2Cel
-1.238mW
-19.7Cel
-1.206mW
-5.4Cel
-1.080mW
32.8mJ/mg
-11.4Cel
-3.843mW
Water Content 45.6wt%
Temperature (℃)
DSC (mW)
0.000
-0.500
-1.000
-1.500
-2.000
-2.500
-3.000
-3.500
-4.000
-100.0
-80.0
-60.0
-40.0
-20.0
0.0
20.0
40.0

FIG. 5

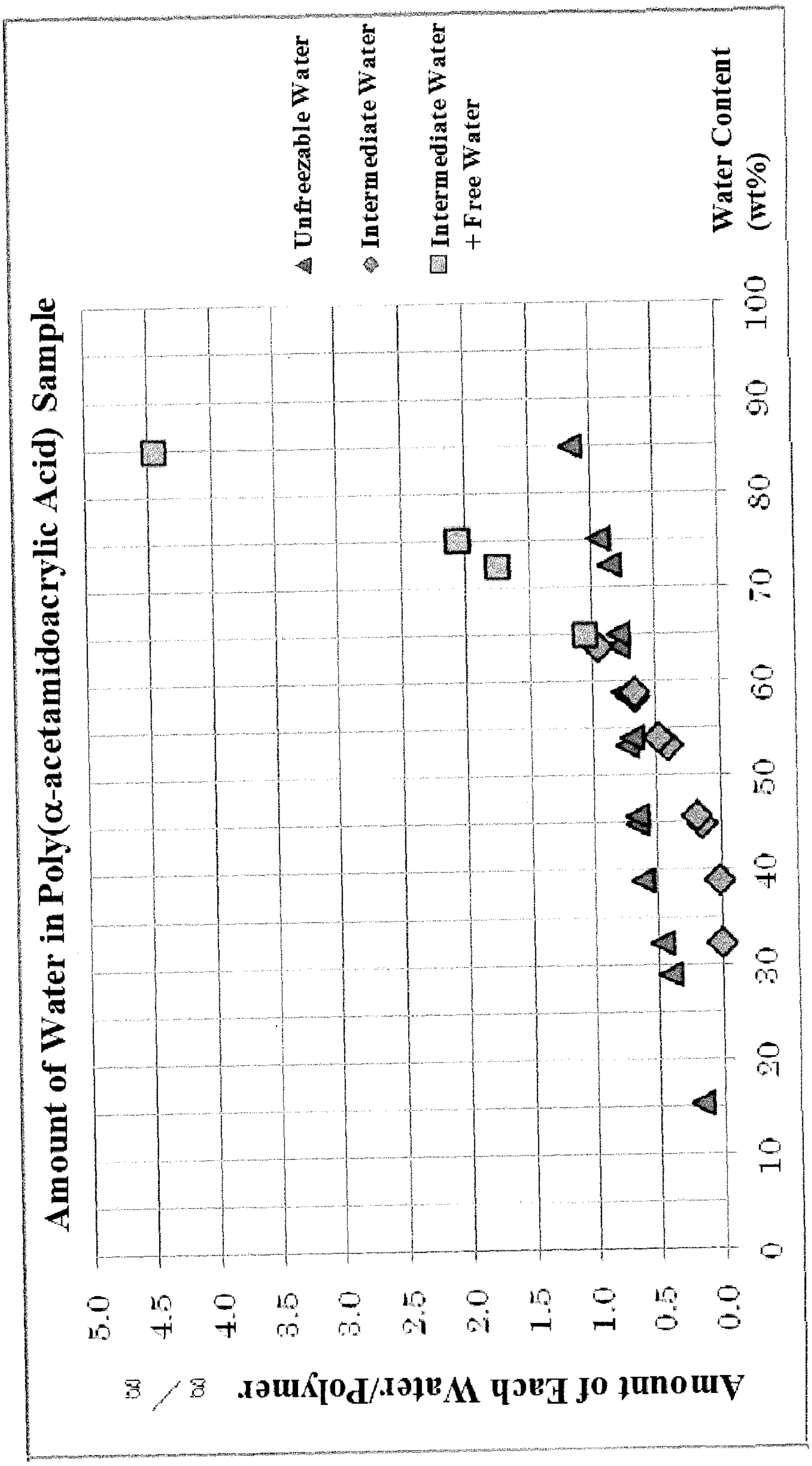
Amount of Water in Poly(α-acetamidoacrylic Acid) Sample
Unfreezable Water
Intermediate Water
Intermediate Water + Free Water
Water Content (wt%)
Amount of Each Water/Polymer g/g
0
10
20
30
40
50
60
70
80
90
100
0.0
0.5
1.0
1.5
2.0
2.5
3.0
3.5
4.0
4.5
5.0

FIG. 6

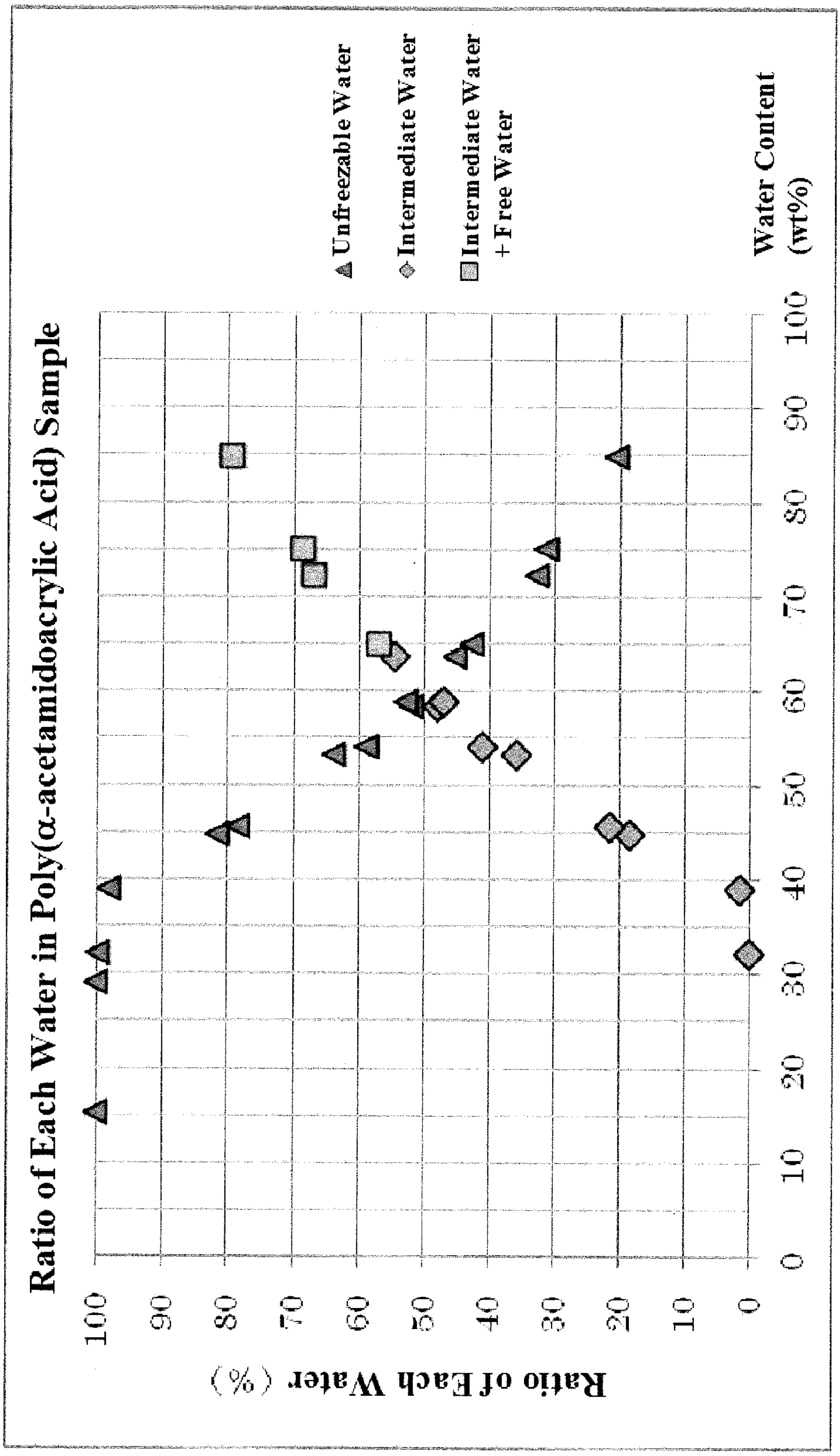
Ratio of Each Water in Poly(α-acetamidoacrylic Acid) Sample
Unfreezable Water
Intermediate Water
Intermediate Water + Free Water
Water Content (wt%)
Ratio of Each Water (%)
0
10
20
30
40
50
60
70
80
90
100

**INTERNATIONAL SEARCH REPORT**

International application No.
PCT/JP2015/058704

A. CLASSIFICATION OF SUBJECT MATTER
*C08F26/02*(2006.01)i, *A61L29/00*(2006.01)i, *A61L31/00*(2006.01)i, *C08L39/00* (2006.01)i

According to International Patent Classification (IPC) or to both national classification and IPC

B. FIELDS SEARCHED

Minimum documentation searched (classification system followed by classification symbols)
C08F26/02, A61L29/00, A61L31/00, C08L39/00

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2015 |
| Kokai Jitsuyo Shinan Koho | 1971-2015 | Toroku Jitsuyo Shinan Koho | 1994-2015 |

Electronic data base consulted during the international search (name of data base and, where practicable, search terms used)
CAplus/REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | US 2626944 A (Coover,Harry W.; Dickey,Joseph B.),<br>09 December 1949 (09.12.1949),<br>claims 1 to 5; page 1, left column, lines 45 to 53<br>(Family: none) | 1-4 |
| X<br>A | JP 48-079782 A (Mitsubishi Rayon Co., Ltd.),<br>25 October 1973 (25.10.1973),<br>claim 1; page 6, upper left column, line 18 to upper right column, line 17<br>(Family: none) | 1-3<br>4 |
| A | JP 2007-126490 A (The University of Tokushima),<br>24 May 2007 (24.05.2007),<br>claim 1<br>(Family: none) | 1-4 |

☒ Further documents are listed in the continuation of Box C.  ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 03 June 2015 (03.06.15) | 16 June 2015 (16.06.15) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (July 2009)

**INTERNATIONAL SEARCH REPORT**

International application No.
PCT/JP2015/058704

C (Continuation). DOCUMENTS CONSIDERED TO BE RELEVANT

| Category* | Citation of document, with indication, where appropriate, of the relevant passages | Relevant to claim No. |
|---|---|---|
| A | JP 2001-064472 A (Showa Denko Kabushiki Kaisha), 13 March 2001 (13.03.2001), claim 1 (Family: none) | 1-4 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2002105136 A
- JP 2004161954 A


### Non-patent literature cited in the description


- Baiomateriaru no Kiso. 2011
- **TSURUTA, T.** Contemporary topics in polymeric materials for biomedical applications. *ADVANCED POLYMER SCIENCE,* 1996, vol. 126, 1-51
- **MORI, Y. ; NAGAOKA, S. ; TAKIGUCHI, T. ; KIKUCHI, T. ; NOGUCHI, N. ; TANZAWA, H. ; NOISHIKI, Y.** A New Antithrombogenic Material with Long Polyethylene Oxide Chains. *Journal of American Society of Artificial Organs,* 1982, vol. 28, 459-463
- Poly(ethylene glycol) chemistry, Biotechnical and Biomedical Application, (U.S.A.). Plenum Press, 1992